# EUROPÄISCHE PATENTSCHRIFT

(11) **EP 1 109 935 B1**
(45) Veröffentlichungstag und Bekanntmachung des Hinweises auf die Patenterteilung: **20.02.2008**
(21) Anmeldenummer: 99944522.4
(22) Anmeldetag: 26.08.1999
(51) Int. Cl.: C12Q 1/68

(54) **ZWEI FARBEN DIFFERENTIAL DISPLAY ALS VERFAHREN ZUR DETEKTION REGULIERTER GENE**
TWO-COLOUR DIFFERENTIAL DISPLAY, USED AS A METHOD FOR DETECTING REGULATED GENES
REPRESENTATION DIFFERENTIELLE EN DEUX COULEURS UTILISE COMME PROCEDE DE DETECTION DE GENES REGULES

(30) Priorität: 07.09.1998 DE 19840731
(43) Veröffentlichungstag der Anmeldung: 27.06.2001
(73) Patentinhaber: Sanofi-Aventis Deutschland GmbH, 65929 Frankfurt am Main (DE)
(72) Erfinder: KOZIAN, Detlef, D-81243 München (DE); REUNER, Birgit, D-81373 München (DE)
(86) Internationale Anmeldenummer: PCT/EP1999/006270
(87) Internationale Veröffentlichungsnummer: WO 2000/014275

(56) Entgegenhaltungen:
- JONES S W ET AL: "Generation of multiple mRNA fingerprints using fluorescence -based differential display and an automated DNA sequencer." BIOTECHNIQUES, (1997 MAR) 22 (3) 536-40, 542-3. , XP002130823 in der Anmeldung erwähnt
- LUEHRSEN K R ET AL: "Analysis of differential display RT-PCR products using fluorescent-- primers and GENESCAN software." BIOTECHNIQUES, (1997 JAN) 22 (1) 168-74. , XP002130824
- SMITH, N. R. ET AL: "Automated differential display using a fluorescently labeled universal primer" BIOTECHNIQUES (1997), 23(2), 274-279 , XP002130825 in der Anmeldung erwähnt
- BAUER ET AL: "IDENTIFICATION OF DIFFERENTIALLY EXPRESSED mRNA SPECIES BY AN IMPROVED DISPLAY TECHNIQUE" NUCLEIC ACIDS RESEARCH,GB,OXFORD UNIVERSITY PRESS, SURREY, Bd. 21, Nr. 18, 11. September 1993 (1993-09-11), Seiten 4272-4280, XP002086977 ISSN: 0305-1048

## Beschreibung

Die Erfindung betrifft ein neues Verfahren zur Analyse der Zusammensetzung einer mRNA Probe und zur Analyse der differentiellen Genexpression, wobei zwei unterschiedlich markierte Primer verwendet werden sowie die Verwendung des Verfahrens.

Differentielles RNA Display (Differential Display: DD) ist eine der am häufigsten angewendeten Methoden zur Detektion und Isolierung regulierter Gene (Liang, P. and Pardee, A. B. (1992) Science 257, 967-971; Liang and Pardee (1995) Current Opinion in Immunology 7, 274-280; McClelland et al. (1995) Trends in Genetics 11: 242-246). Die Methode des DDRT (Differential Display + Reverse Transkription: DDRT) beinhaltet, daß in einem ersten Schritt isolierte RNA unter Verwendung eines ersten Primers revers transkribiert wird (Reverse Transkription (RT)), wobei der erste Strang einer komplementären DNA (cDNA) hergestellt wird und diese dann in einem zweiten Schritt unter Verwendung des ersten Primers und eines zweiten Primers mit der Polymerase Kettenreaktion (PCR) amplifiziert und anschließend die Zusammensetzung der amplifizierten cDNA Probe analysiert wird, beispielsweise durch Auftrennung der amplifizierten cDNA in einem Gel. Der Nachweis kann dann beispielsweise durch Hybridsierung mit einer markierten Probe erfolgen oder durch Markierung der amplifizierten cDNA, aber auch dadurch, daß die Amplifikation in Gegenwart von radioaktiv markierten Nukleotiden (meist radioaktiv markiertes dATP) oder in Gegenwart eines markierten Primers, der z.B. mit einem Fluoreszenzfarbstoff markiert ist ("Fluoreszenz DDRT") (Ito et al. (1994) FEBS Letters 351, 231-236; Ito and Sakaki ("Methods in Molecular Biology Vol. 85: Differential Display Methods and Protocols" (1997) p. 37-44 Liang and Pardee eds, Human Press Inc. Totowa, NJ; Jones et al. (1997) Biotechniques 22, 536-543; Smith et al. (1997) Biotechniques 23, 274-279) durchgeführt wird.

Wird die Reaktion in Gegenwart von radioaktiv markierten Nukleotiden durchgeführt, so werden alle amplifizierten cDNAs markiert (radioaktiver DDRT; klassischer DDRT). Wird hingegen ein markierter Primer verwendet (z.B. Fluoreszenz DDRT), so wird durch die möglichen Primerkombinationen des ersten markierten Primers und des unmarkierten zweiten Primers während der PCR nur ein Teil der amplifizierten cDNAs markiert, während ein anderer Teil unmarkiert bleiben.

Für die RT wird der erste markierte Primer (hier ist Primer 1 ein Oligo(dT)-Primer mit zwei weiteren Nukleotiden (M=A, C, G; N=A, C, G, T) am 3'-Ende ("5'-(T)₁₂-MN-3'" ist (T)₁₂-MN)) eingesetzt. In der anschließenden PCR wird zusätzlich zu diesem ersten Primer ein zweiter unmarkierter Primer (hier ist Primer 2 ein Oligonukleotid mit einer Sequenz aus 10 Nukleotiden die eine zufällige Sequenz aufweisen ("10mer")) eingesetzt, allerdings wird der erste Primer, üblicherweise in einem zweifachen Überschuß eingesetzt. Auf diese Weise wird i.d.R. ein relativ hoher Anteil an amplifizierten cDNAs erhalten, deren 3'-Enden die Sequenz des ersten Primers aufweisen und deren 5'-Enden die Sequenz des zweiten Primers aufweisen (z.B. 5'-10mer ----- (T)₁₂-MN-3', wobei "-----" die zwischen den Primer Sequenzen liegende, amplifizierte cDNA Sequenz symbolisiert). Darüber hinaus werden cDNAs amplifiziert, bei denen die Primer Sequenzen gegenüber 1) vertauscht sind (an deren 5'-Enden die Sequenz des (T)₁₂-MN Primers und an deren 3'-Enden die Sequenz des 10mer-Primers zu finden ist; vergl. 2)). Weitere cDNAs werden entweder nur über den (T)₁₂-MN Primer (vergl. 3) oder nur über den 10mer Primer (vergl. 4) amplifiziert. Bei der Verwendung von zwei verschiedenen Primern können also durch die möglichen Primerkombinationen im DDRT folgende Reaktionsprodukte amplifiziert werden:
1) 5'-10mer --------- (T)₁₂-MN-3'
2) 5'-(T)₁₂-MN --------- 10mer-3'
3) 5'-(T)₁₂-MN ---------- (T)₁₂-MN-3'
4) 5'-10mer ---------10mer-3'

Aufgrund der Tatsache, daß in diesem Fluoreszenz DDRT Ansatz nur der erste Primer markiert ist, werden in der anschließenden Analyse der amplifizierten cDNAs nur diejenigen cDNAs nachgewiesen, die in mindestens einer Richtung über den markierten ersten Primer amplifiziert wurden, d.h. die unter 1), 2) und 3) genannten cDNAs, wogegen cDNAs, die nur mit dem zweiten Primer amplifiziert werden, weder markiert noch nachgewiesen werden (vergl. 4)). Im Vergleich zu einem radioaktiven DDRT ist die Komplexität der nachgewiesenen PCR-Produkte (cDNAs) somit geringer. Aus diesem Grund werden mit dem herkömmlichen Fluoreszenz DDRT diejenigen der regulierten Gene bzw. die dazu korrespondierenden mRNAs nicht erfaßt, die nur mit dem zweiten Primer amplifiziert werden. Somit kann die Anzahl regulierter Gene, die mit dem herkömmlichen Fluoreszenz DDRT erfaßt wird geringer sein, als die im radioaktiven DDRT erfaßte.

Weiterhin wird sowohl mit dem herkömmlichen radioaktiven DDRT als auch mit dem herkömmlichen Fluoreszenz DDRT, wie unter 1) bis 4) dargestellt, ausgehend von einer einzigen mRNA nicht ein einheitliches cDNA Produkt in der PCR amplifiziert, sondern es können durch die verschiedenen Primerkombinationen mehrere cDNAs amplifiziert werden, die sich in ihrer Länge unterscheiden (dies würde z.B. durch Auftrennung in einem Gel sichtbar). Ob es sich bei verschiedenen amplifizierten cDNAs um cDNA Produkte ein und desselben regulierten Gens handelt oder nicht, würde erst eine detaillierte Analyse (z.B. eine Sequenzanalyse) ergeben. Die herkömmlichen DDRTs bieten nicht die Möglichkeit, zwischen den renundanten markierten amplifizierten cDNAs zu unterscheiden. Im radioaktiven DDRT kann zwischen 1) bis 4) nicht unterschieden werden. Im herkömmlichen Fluoreszenz DDRT kann zwischen 1),2) und 3) nicht unterschieden werden, obwohl eine höhere Wahrscheinlichkeit besteht, daß das sichtbare cDNA Fragment aus dem 3'-Bereich eines Gens stammt, da der 3'-Primer mit dem Fluoreszenz-Farbstoff markiert ist.

Somit stellt einerseits die Redundanz von amplifizierten cDNA-Fragmenten im herkömmlichen radioaktiven DDRT, die nicht bzw. nur mit relativ großen Aufwand festgestellt werden kann und andererseits die geringere Komplexität des amplifizierten cDNA Produkts beim herkömmlichen Fluoreszenz DDRT im Vergleich zur herkömmlichen radioaktiven DDRT, mit der Gefahr, bestimmte regulierte Gene zu übersehen, Hauptprobleme der herkömmlichen DDRT bzw. Fluoreszenz DDRT Methode dar.

Die der vorliegenden Anmeldung zugrunde liegende Erfindung stellt ein Verfahren bereit, das zur Analyse von RNA Proben und insbesondere zu Analyse von differentiell regulierten Genen verwendet werden kann, wobei dieses Verfahren die genannten Nachteile nicht aufweist.

Die vorliegende Erfindung betrifft ein Verfahren zur Analyse einer RNA Probe, vorzugsweise einer mRNA Probe, wobei
a) ausgehend von einer RNA Probe, vorzugsweise einer mRNA Probe unter Verwendung eines ersten Primers, der gegebenenfalls mit einem ersten Farbstoff markiert ist, der erste Strang einer komplementären DNA-Probe (cDNA Probe) hergestellt wird,
b) der zweite Strang dieser cDNA Probe unter Verwendung eines zweiten Primers, der vorzugsweise mit einem zweiten Farbstoff markiert ist, hergestellt wird,
c) die cDNA Probe unter Verwendung des ersten Primers, der mit einem ersten Farbstoff markiert ist und des zweiten Primers, der mit einem zweiten Farbstoff markiert ist, amplifiziert wird und
d) die Zusammensetzung der amplifizierten und markierten cDNA Probe analysiert wird.

Die RNA Probe enthält zu analysierende mRNA. In einer bevorzugten Ausführungsform der Erfindung wird in dem Verfahren mRNA eingesetzt. Die RNA kann beispielsweise mit Hilfe von bekannten Verfahren wie CsCl₂-Dichtegradientenzentrifguation oder Säulenchromatographie isoliert werden. Vorzugsweise wird die RNA aus einer Zelle bzw. einer Population von Zellen bzw. aus Gewebe isoliert. Gegebenenfalls kann aus der RNA die mRNA angereichert werden, beispielsweise durch Chromatographie über eine Oligo(dT)-Säule.

In dem Verfahren werden ein erster und ein zweiter Primer eingesetzt. Der erste Primer ist der Primer, der für die Reverse Transkription verwendet wird. Der erste Primer kann auch als 3'-Primer bezeichnet werden, da er mit der mRNA hybridisiert und das 3'-Ende des ersten Stranges der komplementären DNA (cDNA) definiert. Der zweite Primer wird benutzt, um den zweiten Strang der cDNA zu synthetisieren. Der zweite Primer wird auch als 5'-Primer bezeichnet, da er mit dem ersten Strang der cDNA hybridisiert und das 5'-Ende des zweiten Stranges der cDNA definiert.

Der erste Primer und der zweite Primer sind gegebenfalls mit einem Farbstoff markiert. Der erste Primer wird für die Reverse Transkription (Verfahrensschritt a)) und für die anschließende Amplifikation der cDNA (Verfahrensschritt c)) eingesetzt. Der erste Primer, der für die RT eingesetzt wird ist entweder mit einem Farbstoff markiert oder nicht mit einem Farbstoff markiert, letzteres ist z.B. der Fall, wenn die verwendete Reverse Transkriptase, den markierten Primer nicht akzeptiert. Der erste Primer ist in jedem Fall markiert, wenn er für die Amplifikation der cDNA eingesetzt wird. Der erste Primer, der für die RT eingesetzt wird und der erste Primer, der für die Amplifikation eingesetzt wird, haben vorzugsweise die gleiche Sequenz.

Der zweite Primer, der sowohl für die Synthese des zweiten Strangs der cDNA (Verfahrensschritt b)) als auch für die Amplifikation der cDNA (Verfahrensschritt c)) verwendet wird, ist vorzugsweise sowohl während der Zweitstrang-Synthese als auch während der Amplifikation markiert. Vorzugsweise hat der zweite Primer in beiden Fällen die gleiche Sequenz. Vorzugsweise sind Zweitstrangsynthese und Amplifkation der cDNA Bestandteil einer Reaktion, so daß der zweite Primer identisch ist.

In bevorzugten Ausführungsformen der Erfindung sind der erste und der zweite Primer Oligodeoxynukleotide, die aus Deoxadenosin (dA), Deoxyguanosin (dG), Deoxyinosin (dl), Deoxyuridin (dU), (Deoxy-)Thymidin (dT) und Deoxycytidin (dC) aufgebaut sind.

Der erste und/oder der zweite Primer können eine Basensequenz (Sequenz von Adenin, Guanin, Cytosin, Thymin; im folgenden "Sequenz") aufweisen, die komplementär zu der (bzw. den) Sequenz(en) einer (oder mehrerer) bestimmte(n)(r) Nukleinsäuren ist. Ein Primer, der komplementär zu einer bestimmten Sequenz ist, kann mit einer Nukleinsäure, die diese komplementäre Sequenz oder eine Sequenz enthält, die sich von dieser komplementären Sequenz ableitet (also eine gewisse Homologie zu dieser komplementären Sequenz aufweist) hybridisieren. Beispielsweise kann der erste Primer vorzugsweise mit der mRNA hybridisieren. Durch Primerextension wird der erste Strang der cDNA erzeugt. Ein Primer kann auch eine Sequenz, die zu der Sequenz einer bestimmten Nukleinsäure identisch bzw. im wesentlichen identisch ist, aufweisen. Dieser Primer kann dann (unter bestimmten Reaktionsbedingungen) mit einer Nukleinsäure, die eine zu der bestimmten Nukleinsäure komplementäre Sequenz aufweist, hybridisieren. Beispielsweise leitet sich die Sequenz des zweiten Primers von der Sequenz der mRNA ab, d.h. die Sequenz des zweiten Primers kann identisch bzw. im wesentlichen identisch zur mRNA Sequenz sein. Der zweite Primer kann deshalb (unter bestimmten Reaktionsbedingungen) mit einer Nukleinsäure, die eine Sequenz aufweist, die komplementär zu der Sequenz der mRNA ist, z.B. dem ersten Strang der cDNA, hybridisieren.

Die Reaktionsbedingungen unter denen der erste und der zweite Primer mit einer Nukleinsäure hybridisieren, sind vorzugsweise bestimmt durch Temperatur- und/oder Pufferbedingungen. Die Temperaturbedingungen werden vorzugsweise so gewählt, daß die Primer spezifisch mit der komplementären Nukleinsäure Sequenz hybridisieren, d.h. daß überwiegend "korrekte" Basenpaarungen stattfinden (A-T; G-C) und möglichst wenig fehlerhafte Basenpaarungen, d.h. möglichst wird eine "optimale" Hybridisierungstemperatur (Annealingtemperatur) gewählt (z.B. Sambrook et al. (1989) Cold Spring Harbor Laboratory Press). Darüber hinaus werden bestimmte Salzkonzentrationen, insbesondere Mg²⁺-Konzentrationen gewählt.

Eine Nukleinsäure, von deren Sequenz sich die Sequenz des ersten und/oder zweiten Primers ableitet (d.h. zu der diese komplementär oder identisch bzw. im wesentlichen identisch sind), kann beispielsweise eine DNA, z.B. eine cDNA, ein Gen oder ein Teil davon oder eine RNA, vorzugsweise eine mRNA sein.

Der zweite Primer kann eine zufällige Basensequenz aufweisen, wobei dies sowohl Primer beinhaltet die eine vollständig zufällige Sequenz aufweisen, als auch solche Primer, die eine teilweise zufällige Sequenz aufweisen (z.B. zufällig im Bezug auf eine oder mehrere Basen). Die Sequenzen können zufällig sein, dabei aber ein bestimmtes Verhältnis der einzelnen Basen aufweisen, z.B. können alle Basen im gleichen Verhältnis vorkommen oder eine oder mehrere Basen sind über- bzw. unterrepräsentiert. Insbesondere kann auch Hypoxanthin verwendet werden.

Der zweite Primer kann auch ein oder mehrere konkrete Basensequenzen aufweisen. Diese konkrete(n) Sequenze(n) kann/können zufällig ausgewählt sein, d.h. sie leiten sich nicht von bekannten Sequenzen ab. Mit solchen Primern können neue, bisher nicht identifizierte Gene bzw. Proteine identifiziert werden.

Der zweite Primer kann eine Basensequenz aufweisen, die sich von bekannten Sequenzen ableitet, d.h. die ganz oder teilweise bekannten Sequenzen entsprechen (d.h. zu diesen Sequenzen komplementär ist oder mit diesen identisch bzw. im wesentlichen identisch ist). Beispielsweise kann ein Primer eine Sequenz aufweisen, die eine mehr oder weniger große Übereinstimmung mit einer bestimmten Konsensus-Sequenz aufweist bzw. dieser entspricht. Auf diese Weise kann beispielsweise die differentielle Expression von bekannten und/oder unbekannten Mitgliedern einer bestimmten Genfamilie/Proteinfamilie identifiziert werden.

Der zweite Primer kann eine Basensequenz aufweisen die sich von einer bestimmten Aminosäuresequenz (z.B. einer Konsensus-Sequenz auf Aminosäure-Ebene) ableitet. In diesem Fall können die Primer degeneriert sein, d.h im Bezug auf eine bestimmte Aminosäure-Sequenz wird die Degeneration des genetischen Codes berücksichtigt; der Primer stellt dann eine Mischung von Primer-Molekülen dar, die eine unterschiedliche Sequenz aufweisen und alle Möglichkeiten der Codon Verwendung berücksichtigen, um für die entsprechende Aminosäure-Sequenz zu kodieren (degenerierter Primer).

Vorzugsweise weist die Sequenz des zweiten Primers eine Restriktionsschnittstelle für eine Restriktionsendonuklease auf.

Der zweite Primer kann vorzugsweise eine Länge von 8 bis 20 Nukleotiden aufweisen, um eine möglichst spezifische Hybridisierung zu gewährleisten. Die Länge wird jedoch vorzugsweise entsprechend den jeweiligen Primer Sequenzen und/oder den Reaktionsbedingungen ausgewählt. Die Länge und Sequenz des ersten und des zweiten Primers werden unabhängig voneinander ausgewählt.

Der erste und/oder der zweite Primer können auch eine Sequenz enthalten, die nicht für die Hybridisierung notwendig sind bzw. die nicht zur Hybridisierung beitragen. Solche anderen Sequenzen können z.B. die weitere Charakterisierung bzw. Verwendung der amplifizierten cDNA ermöglichen bzw. erleichtern. Vorzugsweise sind diese anderen Sequenzen am 5'-Ende des Primers lokalisiert. Beispielsweise kann der Primer eine Sequenz enthalten, die eine anschließende Sequenzierung erleichtert, z.B. eine M13-Sequenz und/oder eine Sequenz, die die Herstellung einer markierten Sonde (z.B. für die Hybridisierung von Northern-Blots, Southern-Blots und/oder für die in situ Hybridisierung) erleichtert, z.B. eine T7-Promotorsequenz, eine T3-Promotorsequenz oder eine SP6-Promotorsequenz.

Im ersten Schritt des Verfahrens wird die mRNA revers transkribiert, wobei der erste Strang einer cDNA hergestellt wird (Verfahrensschritt a)). Für die RT wird ein erster Primer, der gegebenfalls mit einem Farbstoff markiert ist, eingesetzt.
Vorzugsweise weist der erste Primer eine Oligo(dT)-Sequenz ("(T)ₓ", wobei "X" die Anzahl der Thymidin Reste angibt) auf, die mit dem Poly (A)-Schwanz jeder der mRNAs hybridisieren und auf diese Weise ein freies 3'-OH Ende für die Polymerase Reaktion liefern kann. Die Oligo(dT)-Sequenz ist vorzugsweise eine Sequenz aus 10-20 Thymidin Resten (X = 10-20), besonders bevorzugt ist eine Sequenz aus 12 Thymidin Resten (X = 12) bzw. 15 Thymidin Resten (X=15).

In einer besonders bevorzugten Ausführungsform weist die Sequenz des ersten Primers am 3'-Ende der Oligo-(dT)-Sequenz mindestens ein weiteres Nukleotid, vorzugsweise jedoch zwei Nukleotide auf, die nicht zur Oligo(dT)-Sequenz gehören, d.h. daß das erste Nukleotid, das sich an das 3'-Ende der Oligo(dT)-Sequenz anschließt, ist von Thymidin verschieden. Vorzugsweise stellt der erste Primer eine Mischung von Primer-Molekülen dar, die sich in der Sequenz der Nukleotide, die nicht zur Oligo(dT)-Sequenz gehören, unterscheiden. Beispielsweise kann der erste Primer die Sequenz 5'-(T)ₓMN-3' aufweisen, wobei "M" für A (Base Adenin), C (Base Cytosin) oder G (Base Guanin) und "N" für A, C, G oder T (Base Thymin) steht. Beispielsweise kann X=12 oder 15 sein:
SEQ ID NO. 1: 5'- TTTTTTTTTTTTMN -3' (5'-(T)₁₂-MN-3"'),
SEQ ID NO. 2: 5'- TTTTTTTTTTTTTTTMN -3' (5'-(T)₁₅-MN-3"'),
wobei
- "M": A, C oder G und
- "N": A, C, G oder T ist.

Der erste Primer stellt vorzugsweise eine Mischung verschiedener Primer dar, die sich in der Sequenz, die nicht zur Oligo(dT)-Sequenz gehört, unterscheiden.
5'-T₍ₓ₎MN-3' kann ein Gemisch von Primer Molekülen mit den Sequenzen:
5'-T₍ₓ₎AN-3'
5'-T₍ₓ₎CN-3'
5'-T₍ₓ₎GN-3'
mit N=A,C,G,T sein.
Vorzugsweise ist 5'-T₍ₓ₎MN-3' ein Gemisch von Primer Molekülen mit den Sequenzen:
5'-T₍ₓ₎MA-3'
5'-T₍ₓ₎MC-3'
5'-T₍ₓ₎MG-3'
5'-T₍ₓ₎MT-3'
mit M=A,C,G,
wobei die möglichen Basen A, C und G gleichmäßig in der Mischung repräsentiert sind; beispielsweise ist 5'-(T)ₓMN-3' ein Gemisch von Primer Molekülen mit den Sequenzen: 5'-(T)ₓAG, 5'-(T)ₓ CG, 5'-(T)ₓ GG bzw. 5'-(T)ₓ AT, 5'-(T)ₓ CT, 5'-(T)ₓ GT u.s.w.. Auf diese Weise können Primer für die Amplifikation verschiedener mRNAs unbekannter Sequenz zur Verfügung gestellt werden. Gleichzeitig ist gewährleistet, daß die Primer vorzugsweise mit dem 3'-Ende der komplementären Sequenz (am Poly(A)-Schwanz der mRNA) hybridisieren.

Für die RT wird vorzugsweise eine RNA-abhängige DNA-Polymerase, beispielsweise das Enzym Reverse Transkriptase oder eine andere DNA-abhängige Polymerase mit Reverser Transkriptase Aktivität verwendet, z.B. eine entsprechende temperaturstabile Polymerase, die dann auch zur anschließenden Amplifikation der cDNA (Verfahrensschritt c)) verwendet werden kann. In einer besonderen Ausführungsform der Erfindung ist die Zweitstrang-Synthese der cDNA Bestandteil des Verfahrensschritts zur Amplifikation der cDNA, wobei der Verfahrensschritt zur Amplifikation der cDNA vorzugsweise eine PCR-Reaktion.

Die reverse Transkription kann beispielsweise bei 37°C - 50°C durchgeführt werden, vorzugsweise bei 40°C - 45°C, besonders bevorzugt bei 42°C. Vorzugsweise wird eine Hybridisierungstemperatur verwendet, die eine spezifische Hybridisierung des ersten Primers ermöglicht (möglichst wenig fehlerhafte Basenpaarungen) und die eine genügend hohe Aktivität der Polymerase gewährleistet und mit der möglichst vollständige Transkripte erhalten werden können.

Der zweite Strang der cDNA wird unter Verwendung eines zweiten Primers synthetisiert, der vorzugsweise markiert ist. Der zweite Primer weist bevorzugt eine Länge von mindestens 6 Nukleotiden auf (6 mer), besonders bevorzugt eine Länge von 10 (10mer) bis 20 Nukleotide (20mer). In einer besonderen Ausführungsform der Erfindung weist der zweite Primer eine Länge von 13 Nukleotiden auf ("13mer"). Beispielsweise kann die Sequenz des zweiten Primers "(N)ₓ" sein, wobei "X" 6-20 ist und wobei "N" unabhängig voneinander A, C, G oder T ist.

Bei dem ersten und dem zweiten Primer handelt es sich vorzugsweise um synthetische Oligonukleotide, die beispielsweise käuflich erworben werden können oder mit Hilfe eine bekannten Verfahrens an einer Festphase synthetisiert werden (z.B. Phosphoramidt-Verfahren nach Caruthers et al. (1983) Tetrahedron Letters 24, 245). Die Primer sind vorzugsweise aus den Nukleotiden Deoxyadenosin, Deoxyguanosin, Deoxyinosin, Deoxycytidin und Deoxythymidin aufgebaut.

In einer besonders bevorzugten Ausführungsform des Verfahrens ist die Synthese des zweiten Strangs der cDNAs bereits Bestandteil der PCR-Reaktion und wird unter den Reaktionsbedingungen, unter denen die PCR durchgeführt wird, synthetisiert.

Vorzugsweise sind der erste und der zweite Primer unterschiedlich markiert. Prinzipiell kann jede Art der Markierung verwendet werden, z.B. kann ein Primer an Digoxigenin oder Biotin gekoppelt werden, so daß ein mit Hilfe dieses Primers amplifiziertes cDNA-Molekül z.B. mit einem entsprechenden Antikörper bzw. Enzym nachgewiesen werden kann oder ein Primer kann an eine chemische Verbindung gekoppelt sein, die die Umsetzung eines Substrates nach dessen Zugabe erlaubt, z.B. Atto-Phos System (JBL Scientific, San Luis Obispo, CA, USA) oder ECF-Substrat (Amersham-Pharmacia Biotech, Freiburg, Deutschland).

Besonders bevorzugt ist allerdings die Markierung des ersten und/oder des zweiten Primers mit Fluoreszenz-Farbstoffen. Vorzugsweise wird der erste Primer mit einem ersten Fluoreszenz-Farbstoff markiert und der zweite Primer mit einem zweiten Fluoreszenz-Farbstoff. Vorzugsweise werden der erste und der zweite Fluoreszenz-Farbstoff so gewählt, daß eine deutliche Unterscheidung der beiden verwendeten Fluoreszenz-Farbstoffe möglich ist. Dabei ist die Eindeutigkeit des Ergebnisses d.h. der detektierbare Unterschied individueller Markierungsmuster einzelner cDNAs sowohl von den verwendeten Fluoreszenz-Farbstoffen als auch von der Sensitivität des Analyseverfahrens abhängig. In der Regel werden für die Auswertung computergestützte Analyseverfahren verwendet. Beispielsweise können die Fluoreszenz-Farbstoffe so gewählt werden, daß sich die detektierbaren Anregungswellenlängen und/oder Emissionswellenlängen des ersten und des zweiten Fluoreszenz-Farbstoffs um 200 nm oder mehr unterscheiden, z.B. 250 nm, 300 nm, 350 nm, 400nm.

Beispiele für Fluoreszenz-Farbstoffe, an die der erste und/oder der zweite Primer gekoppelt werden können sind Cy2, Cy3, Cy5, FAM, 6-FAM (6-Carboxy-fluorescein (blau)), FITC (Fluorescein-isothiocyant) , Fluorescein, HEX (4, 5, 2',4',5',7'-Hexacloro-6-carboxyfluorescein (grün)), 5-IAF, TAMRA (6-Carboxytetramethylrhodamine (gelb)), TET (4, 7, 2', 7'-Tetrachloro-6-carboxyfluorescein), XRITC (Rhodamin-X-isothiocyant), ROX (6-Carboxyrhodamin (Rot)), Alexa488, Alexa532, Alexa546, Alexa594, TexasRed und Lissamin.

### Beispielsweise sind folgende Kombinationen möglich:

Primer 1 markiert mit Cy5 und Primer 2 markiert mit Alexa488; Primer 1 markiert mit Cy5 und Primer 2 markiert mit FITC; Primer 1 markiert mit Cy5 und Primer 2 markiert mit FAM; Primer 1 markiert mit Cy5 und Primer 2 markiert mit Cy2; Primer 1 markiert mit Cy5 und Primer 2 markiert mit Cy3; Primer 1 markiert mit Fluorescein und Primer 2 markiert mit Texas Red; Primer 1 markiert mit Fluorescein und Primer 2 markiert mit Lissamin; Primer 1 markiert mit Fluorescein und Primer 2 markiert mit ROX; Primer 1 markiert mit Fluorescein-Cy3; Primer 1 markiert mit Alexa594 und Primer 2 markiert mit Alexa488; Primer 1 markiert mit Alexa568 und Primer 2 markiert mit Alexa488; Primer 1 markiert mit Alexa546 und Primer 2 markiert mit Alexa488 ; Primer 1 markiert mit Alexa532 und Primer 2 markiert mit Alexa488; Primer 1 markiert mit Texas Red und Primer 2 markiert mit Alexa488; Primer 1 markiert mit ROX und Primer 2 markiert mit Alexa488; Primer 1 markiert mit Alexa488 und Primer 2 markiert mit Lissamine; Primer 1 markiert mit Alexa488 und Primer 2 markiert mit Cy3; Primer 1 markiert mit Alexa488 und Primer 2 markiert mit ROX. Natürlich sind auch entsprechende Primerkombinationen möglich, bei denen die zur Markierung verwendeten Fluoreszenz-Farbstoffe im Bezug auf Primer 1 und Primer 2 gegenüber den oben dargelegten Beispielen vertauscht sind.

Vorzugsweise werden die Fluoreszenz-Farbstoffe an das 5'-Ende des Primers (Oligonukleotids) gekoppelt. Im Fall eines Oligo(dT)-Primers kann am 5'-Ende vor dem Fluoreszenz-Farbstoff eine weiteres, von Thymidin verschiedenes Nukleotid lokalisiert sein, beispielsweise ein Guanosin. Darüber hinaus kann ein Fluoreszenz-Farbstoff auch über eine Base an das Oligonukleotid gekoppelt werden. Gegebenenfalls kann ein Fluoreszenz-Farbstoff auch über einen geeigneten Linker an den ersten und/oder den zweiten Primer gekoppelt werden.

Beispiele für markierte Primer, die als erster und/oder zweiter Primer verwendet werden können, sind:
5'- CY5-G(T)₁₅MN -3', 5'- CY2-G(T)₁₅MN -3', 5'- CY3-G(T)₁₅MN -3',
5'- FAM-G(T)₁₅MN -3', 5'- 6-FAM-G(T)₁₅MN -3', 5'- FITC-G(T)₁₅MN -3',
5'- Fluorscein -G(T)₁₅MN -3', 5'- HEX-G(T)₁₅MN -3', 5'- 5-IAF -G(T)₁₅MN -3',
5'- TAMRA-G(T)₁₅MN -3', 5'- TET-G(T)₁₅MN -3', 5'- XRITC -G(T)₁₅MN -3',
5'- ROX-G(T)₁₅MN -3', 5'- Alexa488 -G(T)₁₅MN -3', 5'- Alexa532 -G(T)₁₅MN -3',
5'- Alexa546 -G(T)₁₅MN -3', 5'- Alexa594 -G(T)₁₅MN -3',
5'- TexasRed -G(T)₁₅MN -3', 5'- Lissamin -G(T)₁₅MN -3' (die bisher genannten Primer werden vorzugsweise als erste Primer verwendet), 5'- CY5- (N)₁₃-3',
5'- CY2- (N)₁₃-3', 5'- CY3- (N)₁₃-3', 5'- FAM - (N)₁₃-3', 5'- 6-FAM - (N)₁₃-3',
5'-FITC- (N)₁₃-3', 5'- Fluorscein - (N)₁₃-3', 5'- HEX - (N)₁₃-3', 5'- 5-IAF - (N)₁₃-3',
5'- TAMRA - (N)₁₃-3', 5'- TET - (N)₁₃-3', 5'- XRITC - (N)₁₃-3', 5'- ROX - (N)₁₃-3',
5'- Alexa488- (N)₁₃-3', 5'- Alexa532- (N)₁₃-3', 5'- Alexa546- (N)₁₃-3', 5'- Alexa594-(N)₁₃-3', 5'- TexasRed - (N)₁₃-3'und 5'- Lissamin - (N)₁₃-3' (diese Primer werden vorzugsweise als zweite Primer verwendet),
wobei
"M" A, C oder G und
"N" A, C, G oder T ist.

Bei der Markierung des ersten und/oder des zweiten Primers mit einem Fluoreszenz-Farbstoff ist darauf zu achten, daß die Anregungswellenlängen genügend weit auseinander liegen, um nach der Anregung feststellen zu können, aufgrund welcher Wellenlänge eine Emission zu beobachten war. Eine gute Kombination ist beispielsweise eine Cy5-Markierung (Anregung bei 650nm) z.B. des ersten Primers (z.B. eines TX-MN-Primers) und eine auf Fluorescein basierende Markierung (Anregung bei 490nm) des zweiten Primers. Die Verwendung von zwei unterschiedlich markierten Primern läßt eine eindeutige Aussage über die Primerzusammensetzung einer detektierten amplifizierten cDNA(s) z.B. im Gel zu.

Ist z.B. nur eine Emission bei 650nm zu beobachten, so wurde die cDNA mit dem mit Cy5 markierten Primer amplifiziert, diese cDNA besitzt zumindest an einem Ende den ersten Primer (z.B. den (T)ₓ-MN-Primer). Ist bei einer Anregungswellenlänge von 490nm eine Emission zu beobachten, so ist bei der cDNA mindestens ein Ende mit dem zweiten Primer versehen. Wenn eine bestimmte amplifizierte cDNA (im Gel) nur bei einer Anregungswellenlänge emittiert, so wurde bei der Amplifikation nur die cDNA mit einem Primer und zwar der, der mit einem Fluoreszenz-Farbstoff, der bei der entsprechenden Anregungswellenlänge emittiert, amplifiziert. Wenn eine bestimmte amplifizierte cDNA (im Gel), bei zwei Anregungswellenlänge emittiert, dann wurde diese cDNA unter Verwendung des ersten und/oder des zweiten Primers amplifiziert.

Die Amplifikation (vorzugsweise PCR) wird unter Verwendung einer DNA-Polymerase, vorzugsweise einer DNA-abhängigen DNA-Polymerase durchgeführt, wobei es sich besonders bevorzugt um eine temperaturstabile DNA-Polymerase handelt, z.B. Taq-Polymerase, VENT-Polymerase, AmpliTaq Polymerase, AmpliTaq Gold Polymerase u.a.. Die PCR wird unter Anwendung eines Temperaturprofils durchgeführt, das eine, vorzugsweise exponentielle Amplifikation der cDNA ermöglicht. Beispielsweise kann dazu der in Beispiel 1 aufgeführte Temperaturzyklus verwendet werden. Vorzugsweise werden 30-40 oder mehr Zyklen gefahren.

Im Anschluß daran wird die markierte und amplifizierte cDNA analysiert. Beispielsweise kann die amplifizierte cDNA in einem Gel aufgetrennt werden, wobei amplifizierte cDNAs, die sich in ihrer Länge unterscheiden in unterschiedlichen Banden lokalisiert sind. Die weitere Analyse solcher Banden kann z.B. in Scannern erfolgen, die den Fluoreszenz-Farbstoff bei einer gegebenen Wellenlänge (die vom jeweils verwendeten Fluoreszenz-Farbstoff abhängt) anregen und/oder die das Licht, das bei einer chemischen Umsetzung eines Chemiluminiszenz-Substrates emittiert wird, detektieren können. Das emittierte Fluoreszenzlicht kann dann z.B. mit einem entsprechenden Computerprogramm ausgewertet und zu einem Gelbild zusammengesetzt werden (wie man es z.B. von Autoradiographien kennt). Als Scanner können beispielsweise die Scanner Fluorimager 575, Fluorimager SI, Fluorimager (Molecular Dynamics, Krefeld), Strom (Molecular Dynamics), FLA-2000 (Fuji, Tokio, Japan), FMBioll (Hitachi, über Biozym Diagnostic GmbH, Hess. Oldendorf), Fluor-S-Multilmager und Molecular Imager FX (BioRad, München) verwendet werden.

Insbesondere wird in dem Verfahren mRNA (auch "mRNA Probe"), die aus einer Zelle isoliert wird, eingesetzt. Eine solche Probe ist i.d.R. eine heterogene mRNA Probe (d.h. sie enthält eine Mischung der zum Zeitpunkt der Isolierung der RNA vorhandenen mRNA dieser Zelle). Diese heterogene mRNA Probe repräsentiert in der Regel die von einer bestimmten Zelle zu einem bestimmten Zeitpunkt unter bestimmten Bedingungen exprimierten Gene, d.h. die Zusammensetzung der mRNA Probe hängt beispielsweise vom Zelltyp, Differenzierungsstatus, Zellzyklus und/oder von der vorhergehenden Behandlung der Zelle u.s.w. ab.

Eine besondere Ausführungsform der Erfindung betrifft die Analyse einer heterogenen mRNA Probe, wobei
a) ausgehend von einer heterogenen mRNA Probe unter Verwendung eines ersten Primers, der gegebenenfalls mit einem ersten Farbstoff markiert ist, der erste Strang einer komplementären heterogenen DNA-Probe (heterogene cDNA Probe) hergestellt wird,
b) der zweite Strang dieser heterogenen cDNA Probe unter Verwendung eines zweiten Primers, der vorzugsweise mit einem zweiten Farbstoff markiert ist, hergestellt wird,
c) die heterogene cDNA Probe unter Verwendung des ersten Primers, der mit einem ersten Farbstoff markiert ist ("markierter erster Primer") und des zweiten Primers, der mit einem zweiten Farbstoff markiert ist ("markierter zweiter Primer"), amplifiziert wird und
d) die Zusammensetzung der heterogenen, amplifizierten und markierten cDNA Probe analysiert wird.

Bei den von einer bestimmten Zelle zu einem bestimmten Zeitpunkt exprimierten Genen sind besonders die von Interesse, die differentiell exprimiert bzw. reguliert werden. Insbesondere ist hier die vergleichende Analyse differentiell exprimierter Gene von Interesse. Eine besondere Ausführungsform der Erfindung betrifft ein entsprechend analoges Verfahren zur vergleichenden Analyse von zwei oder mehreren heterogen mRNA Proben.

Eine besondere Ausführungsform der Erfindung betrifft ein Verfahren zur vergleichenden Analyse einer ersten heterogenen mRNA Probe mit einer oder mehreren weiteren heterogenen mRNA Proben, wobei
a) ausgehend von zwei oder mehreren heterogenen mRNA Proben für jede dieser mRNA Proben unter Verwendung eines ersten Primers, der gegebenenfalls mit einem ersten Farbstoff markiert ist, der jeweils erste Strang einer komplementären, heterogenen DNA-Probe (heterogene cDNA Probe) hergestellt wird,
b) dann für jede dieser Proben der jeweils zweite Strang der heterogenen cDNA Probe unter Verwendung eines zweiten Primers, der vorzugsweise mit einem zweiten Farbstoff markiert ist, hergestellt wird,
c) jede der heterogenen cDNA Proben unter Verwendung des ersten markierten Primers und des zweiten markierten Primers amplifiziert wird und
d) die Zusammensetzung jeder heterogenen, amplifizierten und markierten cDNA Probe analysiert und die Zusammensetzung der Proben verglichen werden.

Eine weitere besondere Ausführungsform der Erfindung betrifft ein Verfahren, wobei
a) ausgehend von zwei oder mehreren heterogenen mRNA Proben für jede dieser mRNA Proben unter Verwendung eines ersten Primers, der gegebenenfalls mit einem ersten Farbstoff markiert ist, der jeweils erste Strang einer komplementären, heterogenen DNA-Probe (heterogene cDNA Probe) hergestellt wird,
b) dann für jede dieser Proben der jeweils zweite Strang der heterogenen cDNA Probe unter Verwendung eines zweiten Primers, der vorzugsweise mit einem zweiten Farbstoff markiert ist, hergestellt wird,
c) jede der heterogenen cDNA Proben unter Verwendung des ersten markierten Primers und des zweiten markierten Primers amplifiziert wird und
d) analysiert wird, in welcher oder welchen der Proben individuelle mRNA Moleküle enthalten oder nicht enthalten sind.

Im Anschluß an die Amplifikation der cDNA und gegebenfalls Analyse der Zusammensetzung der cDNA wird bestimmt, welche der amplifizierten cDNAs welche Primerzusammensetzung aufweisen, d.h. welche cDNAs nur mit dem ersten Primer amplifiziert wurden, welche nur mit dem zweiten Primer amplifiziert wurden und welche mit dem ersten und dem zweiten Primer amplifiziert wurden (Zugehörigkeit der differentiell amplifizierten cDNAs zu den Gruppen 1)+2), 3) und 4)). Die Erfindung betrifft auch weitergehende Verfahren, in denen Aufgrund der Primerzusammensetzung amplifizierter cDNAs bestimmte amplifizierte cDNAs ausgewählt und ggf. weiter analysiert und/oder weiter verwendet werden. Die Erfindung betrifft darüber hinaus weitergehende Verfahren, in denen die Verteilung der Primerzusammensetzung der gesamten amplifizierten cDNAs bezüglich der Gruppen 1)-4) analysiert wird.

### Beispiele für weitergehende Verfahren sind:

Ein Verfahren zur Analyse einer RNA Probe, vorzugsweise einer mRNA Probe,
wobei
a) ausgehend von einer RNA Probe, vorzugsweise einer mRNA Probe unter Verwendung eines ersten Primers, der gegebenenfalls mit einem ersten Farbstoff markiert ist, der erste Strang einer komplementären DNA-Probe (cDNA Probe) hergestellt wird,
b) der zweite Strang dieser cDNA Probe unter Verwendung eines zweiten Primers, der vorzugsweise mit einem zweiten Farbstoff markiert ist, hergestellt wird,
c) die cDNA Probe unter Verwendung des ersten Primers, der mit einem ersten Farbstoff markiert ist und des zweiten Primers, der mit einem zweiten Farbstoff markiert ist, amplifiziert und
d) die Zusammensetzung der amplifizierten und markierten cDNA Probe analysiert wird und
e) die Primerzusammensetzung von amplifizierten cDNAs bestimmt wird.

Ein Verfahren zur Analyse einer RNA Probe, vorzugsweise einer mRNA Probe,
wobei
a) ausgehend von einer RNA Probe, vorzugsweise einer mRNA Probe unter Verwendung eines ersten Primers, der gegebenenfalls mit einem ersten Farbstoff markiert ist, der erste Strang einer komplementären DNA-Probe (cDNA Probe) hergestellt wird,
b) der zweite Strang dieser cDNA Probe unter Verwendung eines zweiten Primers, der vorzugsweise mit einem zweiten Farbstoff markiert ist, hergestellt wird,
c) die cDNA Probe unter Verwendung des ersten Primers, der mit einem ersten Farbstoff markiert ist und des zweiten Primers, der mit einem zweiten Farbstoff markiert ist, amplifiziert und
d) die Zusammensetzung der amplifizierten und markierten cDNA Probe analysiert wird,
e) die Primerzusammensetzung von amplifizierten cDNAs bestimmt wird und
f) cDNAs mit einer bestimmten Primerzusammensetzung, vorzugsweise solche, die sowohl den ersten als auch den zweiten Primer enthalten, ausgewählt und weiter analysiert werden.

Die RNA bzw. mRNA Proben, die für die vergleichende Analyse bzw. Verfahren verwendet werden, können z.B. aus verschiedenen Zellen isoliert werden, deren Expressionsmuster miteinander verglichen werden soll. Dabei kann es sich beispielsweise um mRNA Proben, die aus verschiedenen Zellen bzw. Zelltypen stammen, die sich in ihrem Differenzierungs- und/oder Entwicklungsstadium bzw. im Stadium des Zellzyklus unterscheiden oder die eine unterschiedliche Vorgeschichte haben (z.B. unterschiedliche Kulturbedingungen (z.B. pH, Temperatur, Zusammensetzung), die mit pharmakologischen Wirkstoffen oder krankheitsfördernden/-hemmend bzw. -auslösenden Stoffen (z.B. carzinogenen oder mutagenen Stoffen) behandelt bzw. diesen ausgesetzt wurden (z.B. UV-Licht) .....) handeln. Solche RNA bzw. mRNA Proben können mit RNA bzw. mRNA Proben, die diesen Stoffen nicht oder nicht in diesem Maße ausgesetzt wurden, verglichen werden. Die RNA bzw. mRNA Proben können aus bestimmten Geweben isoliert werden. Beispielsweise kann gesundes Gewebe im Vergleich zu pathologischem Gewebe, junges Gewebe im Vergleich zu altem Gewebe, behandeltes Gewebe im Vergleich zu unbehandeltem Gewebe, induziertes Gewebe im Vergleich zu nicht induziertem Gewebe, Gewebe aus verschiedenen Stadien der Entwicklung und/oder des Zellzyklus und/oder der Differenzierung vergleichend analysiert werden, wobei Gewebe für Gewebe, Organ, Zelltyp, Zellen aus Kultur, Zellen einer Zellinie, Zellen eines Individuums steht.

Das Verfahren kann zur Analyse der differentiellen Genexpression (eines Gewebes bzw. einer Zelle) benutzt werden. Insbesondere kann das Verfahren zur vergleichenden Analyse der differentiellen Genexpression (von zwei oder mehreren Geweben bzw. Zellen) verwendet werden.

Das Verfahren kann zur Identifizierung und/oder Charakterisierung von pharmakologischen Wirkstoffen verwendet werden. Weiterhin kann das Verfahren zur Identifizierung und/oder Charakterisierung von Zielgenen bzw. Zielproteinen verwendet werden. Solche Zielgene bzw. Zielproteine sind insbesondere Gene bzw. Proteine, denen eine (möglichst spezifische) Funktion bei der Prävention, der Entstehung und/oder dem Fortschreiten bzw. der Heilung einer Krankheit, beim Differenzierungsprozeß, z.B. der Zelldifferenzierung (ggf. vor bzw. nach Induktion) und/oder beim Differenzierungsprozeß einer Krankheit, im Zellzyklus bzw. der Zellzykluskontrolle und/oder der Zellentwicklung, z.B. dem Zellalterungsprozeß zukommt.

Das Verfahren erweist sich im Gegensatz zu dem herkömmlichen radioaktiven DDRT als vorteilhaft, da die Verwendung von radioaktiv markierten Nukleotiden bzw. radioaktiv markierten Primern durch die Verwendung von Fluoreszenz-markierten Primern ersetzt werden kann. Im Gegensatz zu den bekannten Fluoreszenz DDRTs, wird bei dem hier beschriebenen neuen Verfahren (DDRT Variante) durch die Verwendung von zwei unterschiedlich markierten Primern, vorzugsweise mit fluoreszierenden Farbstoffen unterschiedlicher Anregungsspektren, jede amplifizierte cDNA markiert. Auf diese Weise ist, bei entsprechender Auswertung, die Detektion jeder amplifizierten, markierten cDNA möglich, d.h. die Komplexität der amplifizierten cDNA bleibt erhalten und ist detektierbar (Produkte 1), 2), 3), und 4) sind detektierbar; z.B. Bandenkomplexität im Gel bleibt erhalten). Darüber hinaus sind eindeutige Aussagen über die Primerzusammensetzung einer amplifizierten, markierten cDNA möglich (d.h. Zuordnung zu 1),2),3) und 4) ist möglich). Dadurch wird es möglich, die cDNAs, die weiter analysiert werden sollen aufgrund ihrer Primerzusammensetzung auszuwählen, z.B. amplifizierte cDNAs auszuwählen, die tatsächlich aus dem 3'-Bereich der Gensequenzen stammen. Mit diesem Verfahren, das auf der Verwendung von zwei unterschiedlich markierten Primern basiert, kann deshalb der Aufwand, unter Umständen redundante cDNAs zu isolieren und analysieren reduziert werden. Trotzdem werden gleichzeitig alle amplifizierten cDNAs, wie im herkömmlichen radioaktiven DDRT erfaßt und können analysiert werden, eine Eigenschaft, die im herkömmlichen Fluoreszenz DDRT nicht gegeben ist.

Gegenstand der Erfindung ist auch ein Testkit zur Durchführung des Verfahrens.

### Beispiele:

Die verwendeten Enzyme stammen von Gibco BRL/Life Technologies (Karlsruhe, Deutschland) (Reverse Transkriptase und Taq Polymerase) und Promega (Heidelberg) (RNAsin).
Thermocycler: Perkin Elmer GeneAmp PCR System 2400 (Perkin Elmer, Weiterstadt).

### Beispiel 1: Reverse Transkription

Reaktionsansatz: 1µl RNA (100 ng-1µg Gesamt-RNA bzw. 1ng-10ng poly-A-RNA/mRNA), 1 µl Primer 1 (10 µM Primer 1, z.B. (T)ₓ-MA-3' oder Cy5-(T)ₓMA-3', mit M=A,C,G und X=11-15), 8 µl H₂O (Nuklease frei). Der Reaktionsansatz wurde 5 min bei 70°C und dann auf Eis inkubiert.

Dann wurden 4 µl 5fach RT-Puffer (Gibco BRL), 2 µl DTT [0.1 M], 1 µl SuperScript Reverse Transcriptase [200U/µl] (Gibco BRL), 1 µl RNAsin [40U/µl] und 2 µl dNTP [250µM] zugegeben.

Die Reverse Transkription wurde für 60 min bei 37°-50°C durchgeführt und dann das Enzym bei 70°C (10 min) inaktiviert.

### Beispiel 2: PCR

Zu 2 µl des RT-Ansatzes aus Beispiel1 wurden 2 µl 10fach PCR-Puffer (Gibco BRL), 0.9 µl W-1 Detergenz [1%] (Gibco BRL), 0.75 µl MgCl₂ [50mM] (Gibco BRL), 1,6 µl dNTP [250µM], 0.5 µl Taq DNA-Polymerase [5U/µl] (Gibco BRL), je 1 µl Primer 1 und Primer 2 ([10µM]; z.B. Cy5-T7-(T)ₓ-MA-3' als Primer 1 (wobei "T7" ein Sequenzabschnitt aus dem T7 Promotor ist) und Fluorescein-(N)ₓ mit X=10-25 und N= A, C, G, T), 10,25 µl H₂O gegeben.

Die PCR wurde unter folgenden Bedingungen durchgeführt:
5 min 94°C
40 x: 1 min 94°C, 2 min 40°C-60°C,1 min 72°C
7 min 72°C

Nach abgeschlossener PCR wird der Reaktionsansatz bei 4°C aufbewahrt und dann auf einem Sequenziergel aufgetrennt (5% Polyacrylamid; 8M Harnstoff).

Die markierten amplifizierten cDNAs können z.B. mit einem Scanner, der bei einer Wellenlänge von 430 +/- 30nm (für Fluorescein: Anregungswellenlänge 490 nm, Emissionswellenlänge 520 nm) und 635 +/- 5nm (für Cy5: Anregungswellenlänge 650 nm, Emissionswellenlänge 675 nm) anregt, detektiert werden. Ein Beispiel dafür ist der Molecular Dynamics Storm lmager.

### SEQUENZPROTOKOLL

(1) ALLGEMEINE ANGABEN:
   (i) ANMELDER:
      (A) NAME: Hoechst Marion Roussel Deutschland GmbH
      (B) STRASSE: -
      (C) ORT: Frankfurt
      (D) BUNDESLAND: -
      (E) LAND: Deutschland
      (F) POSTLEITZAHL: 65926
      (G) TELEFON: 069-305-7072
      (H) TELEFAX: 069-35-7175
      (I) TELEX: -
   (ii) BEZEICHNUNG DER ERFINDUNG: Zwei Farben Differential Display als Verfahren zur Detektion regulierter Gene
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 14 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..14
      (D) SONSTIGE ANGABEN:/note= "M = A, C, G; N = A, C, G, T"
   (xi) SEQUENZBESCHREIBUNG: SEQ ID NO: 1:
      TTTTTTTTTT TTMN 14
(2) ANGABEN ZU SEQ ID NO: 2:
   (i) SEQUENZKENNZEICHEN:
      (A) LÄNGE: 17 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: exon
      (B) LAGE:1..17
      (D) SONSTIGE ANGABEN:/note= "M = A, C, G; N = A, C, G, T"
   (xi) SEQUENZBESCHREIBUNG: SEQ ID NO: 2:
      TTTTTTTTTT TTTTTMN

## Patentansprüche

1. Ein Verfahren zur Analyse einer RNA Probe, wobei
a) ausgehend von einer RNA Probe unter Verwendung eines ersten Primers, der gegebenenfalls mit einem ersten Farbstoff markiert ist, der erste Strang einer komplementären DNA-Probe (cDNA Probe) hergestellt wird,
b) der zweite Strang dieser cDNA Probe unter Verwendung eines zweiten Primers, der vorzugsweise mit einem zweiten Farbstoff markiert ist, hergestellt wird,
c) die cDNA Probe unter Verwendung eines ersten Primers, der mit dem ersten Primer aus Schritt a) sequenzidentisch ist, und der mit einem ersten Farbstoff markiert ist und eines zweiten Primers, der mit dem zweiten Primer aus Schritt c) sequenzidentisch ist, und der mit einem zweiten Farbstoff markiert ist, amplifiziert wird und
d) die Zusammensetzung der amplifizierten markierten cDNA Probe analysiert wird.

2. Verfahren nach Anspruch 1, wobei der erste Primer eine Oligo(dT)-Sequenz enthält.

3. Verfahren nach einem der Ansprüche 1 und 2, wobei die Oligo(dT)-Sequenz des ersten Primers aus mindestens 10 Thymidinnukleotiden besteht.

4. Verfahren nach einem der Ansprüche 1 bis 3, wobei der erste Primer am 3'-Ende der Oligo(dT)-Sequenz mindestens zwei weitere Nukleotide enthält, die nicht zur Oligo(dT)-Sequenz gehören.

5. Verfahren nach einem der Ansprüche 1 bis 4, wobei der erste Primer eine heterogene Mischung von Primer-Molekülen darstellt, die sich in der Sequenz der Nukleotide in der Position M oder N, die nicht zur Oligo(dT)-Sequenz gehören, unterscheiden.

6. Verfahren nach einem der Ansprüche 1 bis 5, wobei der zweite Primer eine Länge von mindestens 6 Nukleotiden besitzt.

7. Verfahren nach einem der Ansprüche 1 bis 6, wobei der erste und der zweite Farbstoff unterschiedliche Fluoreszenz-Farbstoffe sind.

8. Verfahren nach einem der Ansprüche 1 bis 7, wobei die Fluoreszenz-Farbstoffe ausgewählt werden aus den Fluoreszenz-Farbstoffen Cy2, Cy3, Cy5, FAM, 6-FAM, FITC, Fluorescein, HEX, 5-IAF, TAMRA, TET, XRITC, ROX, Alexa488, Alexa532, Alexa546, Alexa594, TexasRed und Lissamin.

9. Verfahren nach einem der Ansprüche 1 bis 8, wobei neben einer ersten RNA Probe ein oder mehrere weitere RNA Proben in analoger Weise vergleichend analysiert werden.

10. Verwendung eines Verfahrens nach einem der Ansprüche 1 bis 9 zur Analyse der differentiellen Genexpression.

11. Verwendung eines Verfahrens nach einem der Ansprüche 1 bis 9 zur Identifizierung und/oder Charakterisierung von pharmakologischen Wirkstoffen.

12. Verwendung eines Verfahrens nach einem der Ansprüche 1 bis 9 zur Identifizierung von Zielgenen.

## Claims

1. A method for analyzing an RNA sample, which comprises
a) using a first primer, which is, where appropriate, labeled with a first dye, to prepare the first strand of a complementary DNA sample (cDNA sample) from an RNA sample,
b) using a second primer, which is preferably labeled with a second dye, to prepare the second strand of this cDNA sample,
c) using a first primer, which is identical in sequence to the first primer from step a) and which is labeled with a first dye, and a second primer, which is identical in sequence to the second primer from step b) and which is labeled with a second dye, to amplify the cDNA sample, and
d) analyzing the composition of the amplified, labeled cDNA sample.

2. The method as claimed in claim 1, wherein the first primer contains an oligo (dT) sequence.

3. The method as claimed in one of claims 1 and 2, wherein the oligo (dT) sequence of the first primer is composed of at least 10 thymidine nucleotides.

4. The method as claimed in one of claims 1 to 3, wherein the first primer contains, at the 3' end of the oligo (dT) sequence, at least two further nucleotides, which do not belong to the oligo (dT) sequence.

5. The method as claimed in one of claims 1 to 4, wherein the first primer is a heterogeneous mixture of primer molecules which differ in the sequence of the nucleotides which are in positions M or N and which do not belong to the oligo (dT) sequence.

6. The method as claimed in one of claims 1 to 5, wherein the second primer is at least 6 nucleotides in length.

7. The method as claimed in one of claims 1 to 6, wherein the first and second dyes are different fluorescences.

8. The method as claimed in one of claims 1 to 7, wherein the fluorescences are selected from the fluorescences Cy2, Cy3, Cy5, FAM, 6-FAM, FITC, fluorescein, HEX, 5-IAF, TAMRA, TET, XRITC, ROX, Alexa488, Alexa532, Alexa546, Alexa594, Texas red and lissamine.

9. The method as claimed in one of claims 1 to 8, wherein, apart from a first RNA sample, one or more additional RNA samples are analogously compared analytically.

10. The use of a method as claimed in one of claims 1 to 9 for analyzing differential gene expression.

11. The use of a method as claimed in one of claims 1 to 9 for identifying and/or characterizing pharmacological active compounds.

12. The use of a method as claimed in one of claims 1 to 9 for identifying target genes.

## Revendications

1. Procédé d'analyse d'un échantillon d'ARN dans lequel :
a) en partant d'un échantillon d'ARN en utilisant une première amorce, qui est le cas échéant marquée avec un premier colorant, on prépare le premier brin d'un échantillon d'ADN complémentaire (échantillon cADN);
b) on prépare le deuxième brin de cet échantillon de cADN en utilisant une deuxième amorce, qui est de préférence marquée avec un deuxième colorant;
c) on amplifie l'échantillon de cADN en utilisant une première amorce qui est identique en séquence à la première amorce de l'étape a) et qui est marquée avec un premier colorant et une deuxième amorce qui est identique en séquence avec la deuxième amorce de l'étape b) et qui est marquée avec un deuxième colorant, et
d) on analyse la composition de l'échantillon de cADN marqué amplifié.

2. Procédé selon la revendication 1 dans lequel la première amorce contient une séquence oligo(dT).

3. Procédé selon l'une des revendications 1 et 2 dans lequel la séquence oligo(dT) de la première amorce est constituée d'au moins 10 nucléotides de thymidine.

4. Procédé selon l'une des revendications 1 à 3 dans lequel la première amorce contient, à l'extrémité 3' de la séquence oligo(dT) au moins deux autres nucléotides qui n'appartiennent pas à la séquence oligo(dT).

5. Procédé selon l'une des revendications 1 à 4 dans lequel la première amorce représente un mélange hétérogène de molécules d'amorce qui se différencient dans la séquence des nucléotides dans la position M ou N, qui n'appartiennent pas à la séquence oligo(dT).

6. Procédé selon l'une des revendications 1 à 5 dans lequel la deuxième amorce possède une longueur d'au moins 6 nucléotides.

7. Procédé selon l'une des revendications 1 à 6 dans lequel le premier et le deuxième colorants sont différentes substances de fluorescence.

8. Procédé selon l'une des revendications 1 à 7 dans lequel les colorants de fluorescence sont sélectionnés parmi les colorants de fluorescence Cy2, Cy3, Cy5, FAM, 6-FAM, FITC, fluorescéine, HEX, 5-IAF, TAMRA, TET, XRITC, ROX, Alexa488, Alexa532, Alexa546, Alexa594, TexasRed et Lissamine.

9. Procédé selon l'une des revendications 1 à 8 dans lequel, en plus d'un premier échantillon d'ARN, on effectue une analyse comparative d'un ou de plusieurs autres échantillons d'ARN d'une manière analogue.

10. Utilisation d'un procédé selon l'une des revendications 1 à 9 pour l'analyse de l'expression génétique différentielle.

11. Utilisation d'un procédé selon l'une des revendications 1 à 9 pour l'identification et/ou la caractérisation de principes actifs pharmacologiques.

12. Utilisation d'un procédé selon l'une des revendications 1 à 9 pour l'identification de gènes cibles.
